# EUROPÄISCHE PATENTSCHRIFT

(11) **EP 1 241 157 B1**
(45) Veröffentlichungstag und Bekanntmachung des Hinweises auf die Patenterteilung: **15.09.2004**
(21) Anmeldenummer: 02003429.4
(22) Anmeldetag: 14.02.2002
(51) Int. Cl.: C07C 47/277, C07C 45/38, C07C 69/734, C07C 67/343, C07C 43/315, C07D 319/06, C07D 317/26, C07D 317/30, C07D 317/20

(54) **Verfahren zur Herstellung von 2,7-Dimethyl-2,4,6-octatrienal-monoacetalen**
Process for the preparation of 2,7-dimethyl-2,4,6-octatrienal-monoacetals
Procédé pour la préparation de 2,7-diméthyl-2,4,6-octatriénal-monoacétals

(30) Priorität: 12.03.2001 DE 10112067
(43) Veröffentlichungstag der Anmeldung: 18.09.2002
(73) Patentinhaber: BASF AKTIENGESELLSCHAFT, 67056 Ludwigshafen (DE)
(72) Erfinder: Ernst, Hansgeorg, Dr., 67346 Speyer (DE); Henrich, Klaus, 67454 Hassloch (DE)

(56) Entgegenhaltungen:
- EP-A- 0 112 502
- EP-A- 0 282 760
- ZUMBRUNN, ALBRECHT ET AL: "Synthesen von Carotinen mit psi-Endgruppen und (Z)-Konfiguration an terminalen konjugierten Doppelbindungen" HELV. CHIM. ACTA (1985), 68(6), 1519-39 , XP002201954
- BERNHARD, KURT ET AL: "Synthese von optisch aktiven, natürlichen Carotinoiden und strukturell verwandten Naturprodukten. IX. Synthese von (3R)-Hydroxyechinenon, (3R,3'R)- und (3R,3'S)-Adonixanthin, (3R)-Adonirubin, deren optischen Antipoden und verwandten Verbindungen" HELV. CHIM. ACTA (1981), 64(7), 2469-84 , XP002201955
- JANSEN, F. J. H. M. ET AL: "Synthesis of isotopically labeled carotenoids;investigations on structure and function of carotenoproteins at the atomic level" PURE APPL. CHEM. (1994), 66(5), 963-72 , XP008004519

## Beschreibung

Die Erfindung betrifft ein neues Verfahren zur Herstellung von 2,7-Dimethyl-2,4,6-octatrienal-monoacetalen (im folgenden auch als C₁₀-Dialdehyd-monoacetale bezeichnet) der allgemeinen Formel I, 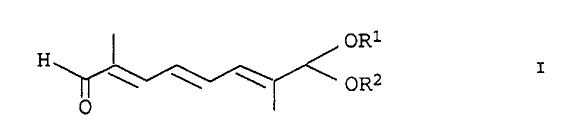 in der die Substituenten R¹ und R² unabhängig voneinander für C₁-C₈-Alkyl stehen oder gemeinsam mit den Sauerstoffatomen und dem Kohlenstoffatom, an denen sie gebunden sind einen 1,3-Dioxolanoder 1,3-Dioxan-Ring der folgenden Strukturen bilden können.

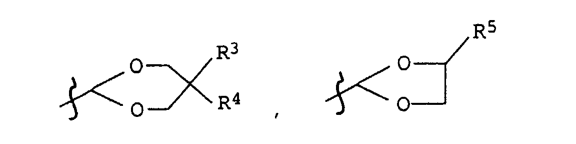

Monoacetale dieser Struktur werden gemäß Helv. Chim. Acta 1981, 64 (7), 2469 für die selektive Synthese unsymmetrisch aufgebauter C₄₀-Carotinoide benötigt.

Der Schutz einer der beiden Carbonylgruppen des zentralen C₁₀-Bausteins (9) im folgenden Reaktionsschema macht es möglich, die Wittig-Reaktionen mit den beiden C₁₅-Phosphoniumsalzen (2) und (5) sehr selektiv nacheinander durchzuführen und so zu einem Carotinoid (6) zu gelangen, das frei von den symmetrisch aufgebauten Produkten (7) bzw. (8) ist, deren Bildung bei Verwendung eines ungeschützten C₁₀-Dialdehyds (9) unvermeidlich ist.

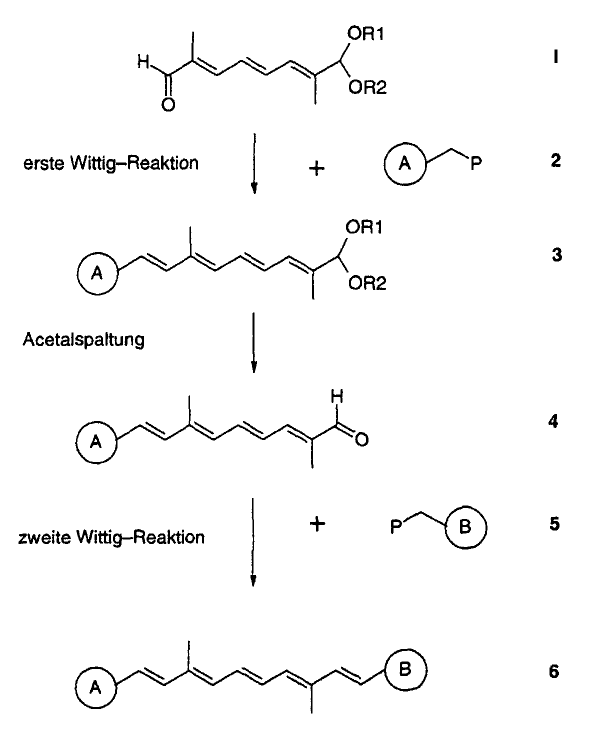

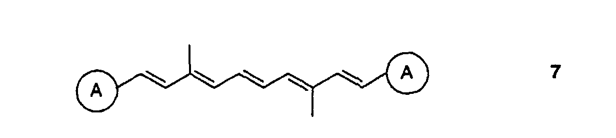

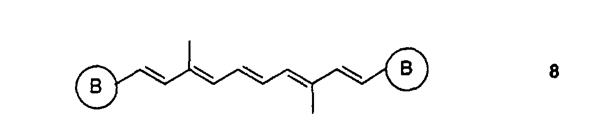

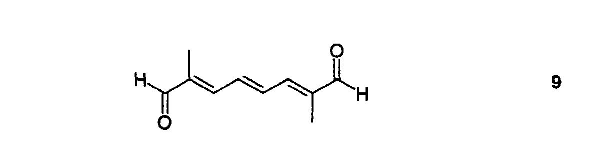

Aus der Gruppe der unsymmetrisch aufgebauten Carotinoide mit unterschiedlichen Endgruppen A und B sind besonders Lutein (10) und meso-Zeaxanthin (11) von Interesse, da diese Carotinoide u.a. das menschliche Auge vor Erblindung als Folge der altersbedingten Makuladegeneration schützen [Exp. Eye Res. 1997, 64 (2), 211-218; GB 2301775 (1996)].

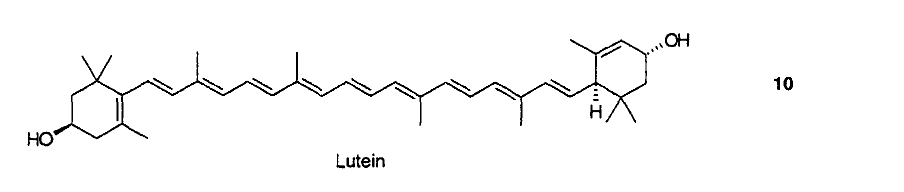

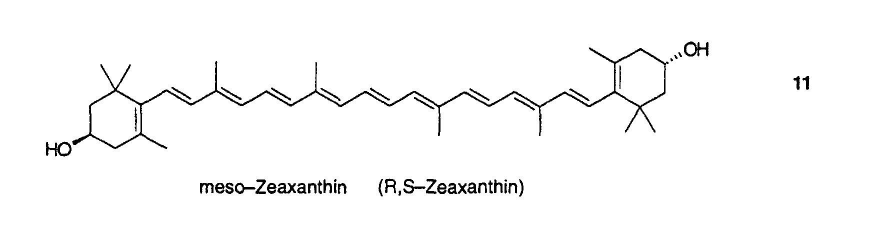

Um meso-Zeaxanthin (11) zu erhalten, das frei von R,R-Zeaxanthin (12) und S,S-Zeaxanthin (13) ist, müssen beispielsweise die beiden Wittig-Reaktionen des zentralen C₁₀-Bausteins mit dem R-C₁₅-Phosphoniumsalz (14) zu einem C₂₅-Zwischenprodukt (15) sowie dessen Umsetzung mit dem S-C₁₅-Phosphoniumsalz (16) nacheinander vollständig selektiv ablaufen.

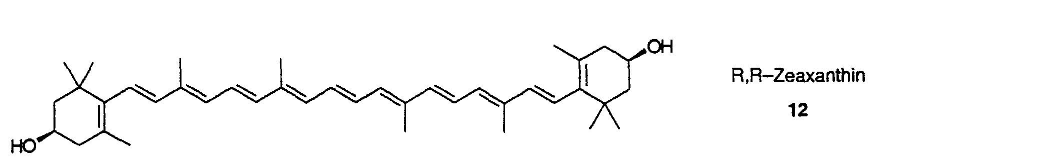

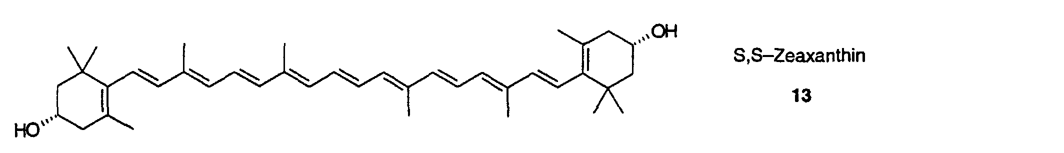

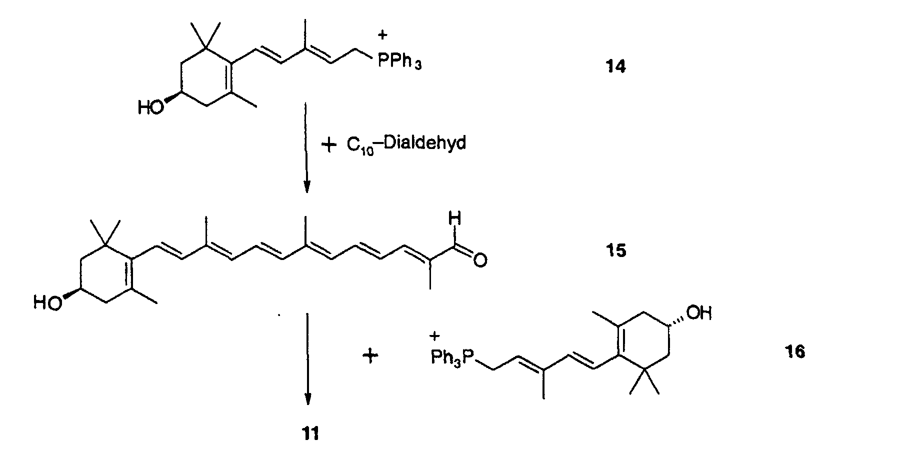

Diese Anforderungen gelten sinngemäß auch für die Synthese von Lutein.

Die für die Synthese eines einheitlichen Produkts erforderliche Selektivität der beiden Wittig-Reaktionen ist nur dann gewährleistet, wenn ein C₁₀-Dialdehyd eingesetzt wird, bei dem eine Carbonylgruppe als Acetal geschützt vorliegt.

Verschiedene Synthesen von C₁₀-Dialdehyd-monoacetalen sind in der Literatur beschrieben worden:

So ist beispielsweise das Dimethylacetal (17) durch p-Toluolsulfonsäure-katalysierte Acetalisierung des Dialdehyds (9) zugänglich [Helv. Chim. Acta 1981, 64 (7), 2469]. Das Verfahren ist gekennzeichnet durch eine aufwendige Tieftemperaturkristallisation und das Produkt ist nur in unzureichender Ausbeute erhältlich.

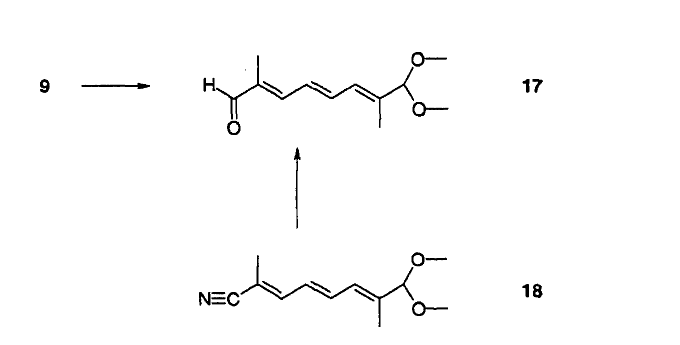

Ein anderes Syntheseverfahren betrifft die Bildung der Aldehydfunktion durch Reduktion des korrespondierenden Nitrils (18) [Pure & Appl. Chem. 1994, 66 (5), 963; Recl. Trav. Chim. Pays-Bas 1994, 113, 552]. Diese Reduktion erfolgt mit Diisobutylaluminiumhydrid bei -70°C. Die gesamte Synthese, die nur im mmol-Maßstab beschrieben ist, hat die Einführung von ¹³C-Isotopen zum Ziel und besitzt keine technische Bedeutung.

Die in der Literatur beschriebene Synthese des C₁₀-Dialdehyd-mononeopentyl-glycolacetals (19) beruht auf der selektiven Spaltung des korrespondierenden Bis-neopentyl-glycolacetals (20) zum Monoacetal durch kurzzeitigen Kontakt mit HCl. Diese Synthese ist ebenfalls nur im mmol-Maßstab beschrieben. Infolge schlechter Selektivität konnte reines Monoacetal (19) erst nach aufwendiger Reinigung durch zweifache Kristallisation in nur 37%iger Ausbeute isoliert werden (Helv. Chim. Acta 1981, 64 (7), 2471). Auch diese Synthese ist für eine technische Realisierung nicht geeignet.

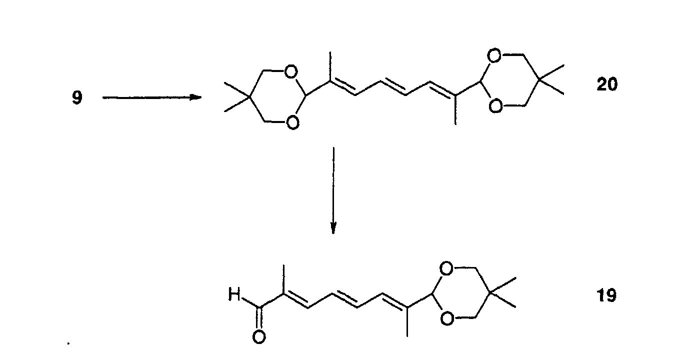

Es war nun die Aufgabe der vorliegenden Erfindung, ein im technischen Maßstab realisierbares und hinsichtlich der Schutzgruppe möglichst flexibles Verfahren zur Herstellung von C₁₀-Dialdehyd-monoacetalen bereitzustellen.

Diese Aufgabe wurde gelöst durch ein Verfahren zur Herstellung von 2,7-Dimethyl-2,4,6-octatrienal-monoacetalen der allgemeinen Formel I, 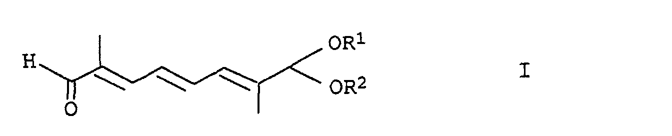 in der die Substituenten R¹ und R² unabhängig voneinander für C₁-C₈-Alkyl stehen oder gemeinsam mit den Sauerstoffatomen und dem Kohlenstoffatom, an denen sie gebunden sind einen 1,3-Dioxolanoder 1,3-Dioxan-Ring der folgenden Strukturen bilden können, 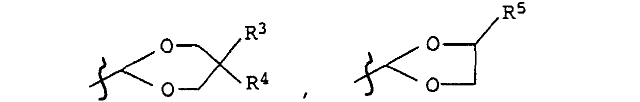 in denen R³ und R⁴ sowie R⁵ jeweils unabhängig voneinander Wasserstoff oder C₁-C₄-Alkyl bedeuten können,
dadurch gekennzeichnet, daß man
a) ein Esterphosphoniumsalz der allgemeinen Formel II oder ein Esterphosphonat der allgemeinen Formel III, 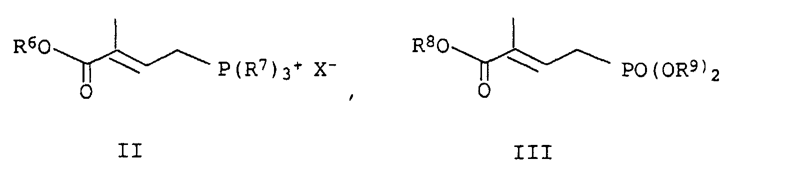 in denen die Substituenten unabhängig voneinander folgende Bedeutung haben:
   - R⁶: C₁-C₈-Alkyl;
   - R⁷: Aryl;
   - X⁻: ein Anionenäquivalent einer anorganischen oder organischen Säure;
   - R⁸ und R⁹: C₁-C₈-Alkyl;
   mit einem Aldehyd der Formel IV 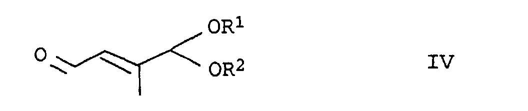 in einer Wittig- oder Wittig-Horner-Reaktion zu einem Acetal-ester der allgemeinen Formel V kondensiert, 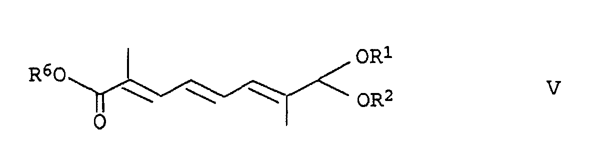 in denen die Substituenten R¹, R² und R⁶ der Verbindungen IV bzw. V die oben genannte Bedeutung haben,
b) den Ester der Formel V zu einem Acetal-alkohol der allgemeinen Formel VI reduziert,
   und 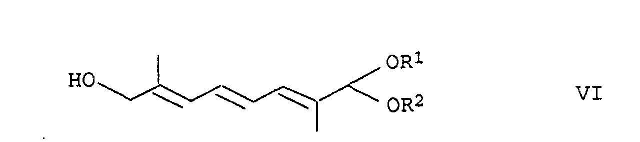
c) den Alkohol zu 2,7-Dimethyl-2,4,6-octatrienal-monoacetalen der allgemeinen Formel I oxidiert.

Überraschenderweise gelang die Lösung dieser Aufgabe in einfacher Weise, ausgehend von C₅-Bausteinen, die in der Polyensynthese etabliert und im technischen Maßstab gut zugänglich sind (vgl. Carotenoids, Vol. 2, "Synthesis", S. 115 ff.; Birkhäuser Verlag 1996).

Im Falle von offenkettigen Acetalen seien als Alkylreste für R¹ und R² lineare oder verzweigte C₁-C₈-Alkylketten, z.B. Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl und n-Octyl genannt.

Bevorzugte Alkylreste für R¹ und R² sind Methyl, Ethyl, n-Propyl und 1-Methylethyl, besonders bevorzugt Methyl und Ethyl.

Als Alkylreste für R³ bis R⁵ seien lineare oder verzweigte C₁-C₄-Alkylketten, z.B. Methyl, Ethyl, n-Propyl, 1-Methylethyl, n-Butyl, 1-Methylpropyl, 2-Methylpropyl und 1,1-Dimethylethyl genannt.

Bevorzugte Reste für R³ bis R⁵ sind Wasserstoff und Methyl.

Als Alkylreste für R⁶, R⁸ und R⁹ seien lineare oder verzweigte C₁-C₈-Alkylketten, z.B. Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl und n-Octyl genannt.

Bevorzugte Alkylreste für R⁶, R⁸ und R⁹ sind Methyl, Ethyl, n-Propyl und 1-Methylethyl, besonders bevorzugt Methyl und Ethyl, ganz besonders bevorzugt Ethyl.

Der Begriff Aryl für R⁷ bezeichnet übliche, in Phosphinen und Phosphoniumsalzen vorkommende Arylreste, wie Phenyl, Toluol, Naphthyl, ggf. jeweils substituiert, bevorzugt Phenyl.

Der Rest X⁻ steht für ein Anionenäquivalent einer anorganischen oder organischen Säure, bevorzugt einer starken anorganischen oder organischen Säure.

Der Ausdruck starke Säure umfaßt Halogenwasserstoffsäuren (insbesondere Salzsäure und Bromwasserstoffsäure), Schwefelsäure, Phosphorsäure, Sulfonsäuren und andere anorganische oder organische Säuren mit vergleichbarem Dissoziationsgrad. Als starke organische Säuren sind in diesem Zusammenhang auch C₁-C₆-Alkansäuren wie Ameisensäure, Essigsäure, Propionsäure, Buttersäure sowie Capronsäure zu verstehen.

Besonders bevorzugt sind Anionen solcher Säure, ausgewählt aus der Gruppe, bestehend aus Salzsäure, Bromwasserstoffsäure, Schwefelsäure, Phosphorsäure, Ameisensäure, Essigsäure und Sulfonsäure zu nennen. Ganz besonders bevorzugt Cl⁻, Br⁻, CₙH₂ₙ₊₁-SO₃- (mit n = 1-4), Ph-SO₃-, p-Tol-SO₃- oder CF₃-SO₃⁻.

Der erste Schritt a) des erfindungsgemäßen Verfahrens beinhaltet die Olefinierungsreaktion eines C₅-Ester-Phosphoniumsalzes der allgemeinen Formel II oder eines C₅-Ester-Phophonats der allgemeinen Formel III mit einem C₅-Acetal-aldehyd der allgemeinen Formel IV, 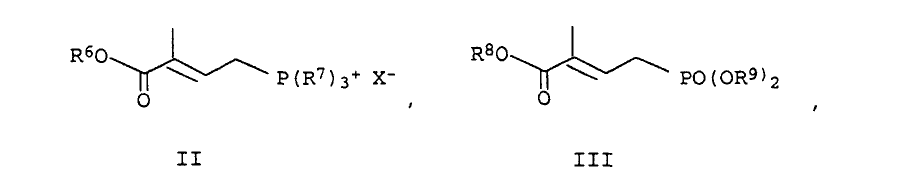 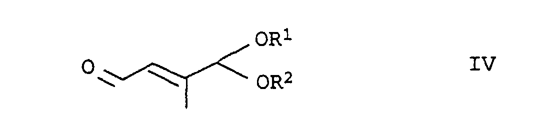 in denen die Substituenten die bereits eingangs genannte Bedeutung haben.

Eine bevorzugte Ausführungsform von Verfahrensschritt a) ist dadurch gekennzeichnet, daß man ein Esterphosphonat der allgemeinen Formel III verwendet, in der die Substituenten R⁸ und R⁹ unabhängig voneinander C₁-C₃-Alkyl, besonders bevorzugt Methyl oder Ethyl, ganz besonders bevorzugt Ethyl bedeuten.

Eine ebenfalls bevorzugte Ausführungsform von Verfahrensschritt a) ist dadurch gekennzeichnet, daß man als Aldehyd eine Verbindung der Formel IVa verwendet, 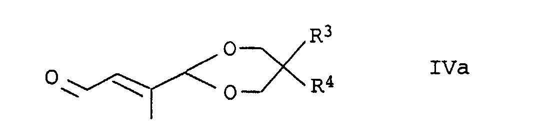 in der die Substituenten R³ und R⁴ unabhängig voneinander Wasserstoff und/oder Methyl, bevorzugt jeweils gemeinsam Wasserstoff oder Methyl, besonders bevorzugt gemeinsam Methyl bedeuten.

Die Kondensation des Phosphoniumsalzes II mit dem Aldehyd IV nach Wittig bzw. des Phosphonats III mit IV nach Wittig-Horner zu einem C₁₀-Acetal-Ester der Formel V wird unter den für diese Reaktionen typischen Bedingungen durchgeführt (siehe Carotenoids, Vol. 2, "Synthesis", S. 79 ff.; Birkhäuser Verlag, 1996, und dort zitierte Literatur).

Die Kondensation von II mit IV kann beispielsweise in einem inerten organischen Lösungsmittel z.B. in offenkettigen oder cyclischen Ethern wie Diethylether, Diisopropylether, Methyltert.butylether, 1,4-Dioxan oder THF, in halogenierten Kohlenwasserstoffen wie Dichlormethan, Chloroform, in aromatischen Kohlenwasserstoffen wie Toluol, Xylol oder Benzol oder in polaren Lösungsmitteln wie Dimethylformamid, Dimethylsulfoxid oder Acetonitril durchgeführt werden. Bevorzugte Lösungsmittel sind Toluol, THF und DMSO oder Mischungen davon.

Als Base können alle für Wittig-Kondensationen üblichen Basen verwendet werden, z.B. Alkalimetallhydroxide wie Natriumhydroxid, Kaliumhydroxid oder Lithiumhydroxid; Alkalimetallhydride wie Natriumhydrid oder Kaliumhydrid.

Als Basen kommen außerdem Lithiumorganyle wie z.B. n-Butyllithium, tert. Butyllithium, Phenyllithium oder Alkalimetallamide wie Lithium-, Kalium- oder Natriumamid, Lithium-diisopropylamid aber auch Alkalimetallhexamethyldisilazide in Frage. Als bevorzugte Base für die erfindungsgemäße Wittig Reaktion werden Natrium- oder Kaliumhexamethyldisilazid sowie Kalium- oder Natriumamid eingesetzt.

Die Menge an eingesetzter Base liegt in der Regel im Bereich von 0,8 bis 5 Mol, bevorzugt 1 bis 3 Mol pro Mol des eingesetzten Phosphoniumsalzes II.

Wenn X⁻ ein Halogenidanion ist, können auch Oxirane vorteilhaft als latente Basen eingesetzt werden (siehe Chem. Ber. 1974, 107, 2050).

Vorzugsweise werden für diese Wittig-Reaktion als Basen Lösungen von Alkalimetallalkoholaten im korrespondierenden Alkohol oder Oxirane, vor allem 1,2-Epoxibutan, ohne zusätzliches Solvens oder im Gemisch mit einem der obengenannten Lösungsmittel oder einem niederen Alkanol verwendet.

Für die Wittig-Horner-Reaktion von III mit IV kommen ebenfalls die für diese Umsetzung typischen Bedingungen zur Anwendung.

Vorzugsweise arbeitet man auch hier in einem der o.g. inerten organischen Lösungsmittel und setzt als Base bevorzugt die Lösung eines Alkalimetallalkoholats im korrespondierenden Alkanol ein. Es ist im Falle der Wittig-Horner-Reaktion aber auch möglich, die oben für die Wittig-Reaktion zusätzlich genannten Basen, mit Ausnahme der Oxirane, zu verwenden.

In den nächsten Verfahrensschritten erfolgt die Umsetzung des Esters V zu dem entsprechenden Aldehyd.

In der Literatur sind verschiedene Methoden beschrieben, Ester direkt in Aldehyde zu überführen. Diese Methoden beinhalten jedoch technisch nicht zugängliche komplexe Hydride wie z.B. Piperazinyl-Aluminiumhydride (Chem. Lett. 1975, 215) oder technisch schwierig zu handhabende Reagenzien wie z.B. Diisobutyl-Aluminiumhydrid, das zudem extrem tiefe Reaktionstemperaturen (-70°C) erfordert.

An ein technisches Verfahren sind aber folgende Anforderungen zu stellen:
- die Umsetzung muß vollständig und mit möglichst hoher Selektivität ablaufen, um aufwendige und teure Trennoperationen zu vermeiden
- extreme Reaktionsbedingungen, vor allem tiefe Temperaturen, sind zu vermeiden
- das Reagens muß in größeren Mengen technisch verfügbar und aus sicherheitstechnischer Sicht gut handhabbar sein.

Aufgrund dieser Vorgaben wird die Estergruppe nach den erfindungsgemäßen Verfahren in einem zweistufigen Prozeß [Stufen b) und c)] in die Aldehydfunktion überführt.

Im Schritt b) wird der Ester der Formel V zunächst zum korrespondierenden Alkohol der Formel VI reduziert.

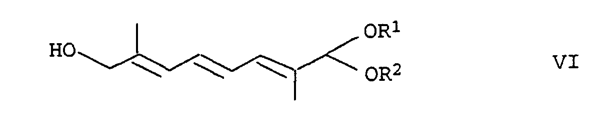

Für diesen Schritt können prinzipiell alle für die Reduktion von Estern zu Alkoholen beschriebenen Hydridreagenzien eingesetzt werden, beispielsweise Alkalimetallbor- oder Alkalimetallaluminiumhydride.

Eine bevorzugte Ausführungsform von Verfahrensschritt b) ist dadurch gekennzeichnet, daß man im die Reduktion der Esterfunktion mit einer Natrium-Aluminium-Hydridverbindung, besonders bevorzugt mit Natrium-dihydro-bis-(2-methoxyethoxy)-aluminat durchführt.

Entsprechend den oben genannten Anforderungen an ein technisches Verfahren ist die handelsübliche konzentrierte toluolische Lösung von Natrium-dihydrido-bis-(2-methoxyethoxy)-aluminat ("Vitride® ") besonders vorteilhaft. Dieses Reagens ist nicht pyrophor, nicht empfindlich gegenüber Sauerstoff (GIT Fachz. Lab. 9/96, 914) und als Flüssigkeit in einem technischen Prozeß wesentlich einfacher handhabbar als feste komplexe Hydride wie z.B. Lithiumaluminiumhydrid.

Die Reaktion wird vorzugsweise so durchgeführt, daß man den Ester der Formel V in einem gegenüber Hydridreagenzien inerten Lösungsmittel wie z.B. Toluol, offenkettigen oder cyclischen Ethern, Glycolethern oder in einem Gemisch dieser Solventien vorlegt und das Reduktionsmittel im Temperaturbereich von -20°C bis 30°C, bevorzugt von -10°C bis 10°C, besonders bevorzugt von -5°C bis 0°C zudosiert.

In der Regel werden pro Äquivalent Ester mindestens 2 Äquivalente Hydrid eingesetzt, d.h. mindestens 0,5 mol Lithiumaluminiumhydrid/mol Ester bzw. 1,0 mol Vitride/mol Ester. Um einen vollständigen Umsatz zu erzielen, ist es jedoch vorteilhaft, einen gewissen Überschuß an Reduktionsmittel einzusetzen. Dieser Überschuß liegt im Bereich von 10 bis 50 mol-%, vorzugsweise 20 bis 30 mol-%.

In der bevorzugten Ausführungsform des erfindungsgemäßen Verfahrens wird der C₁₀-Acetal-ester V mit der toluolischen Vitride-Lösung zum C₁₀-Acetal-alkohol VI reduziert. Nach wäßriger Aufarbeitung erhält man in praktisch quantitativer Ausbeute ein Rohprodukt, das ohne Reinigung direkt in die Folgestufe c) eingesetzt werden kann.

Die Oxidation eines C₁₀-Acetal-alkohols der Formel VIa zum entsprechenden Aldehyd ist in der Literatur bis dato noch nicht beschrieben.

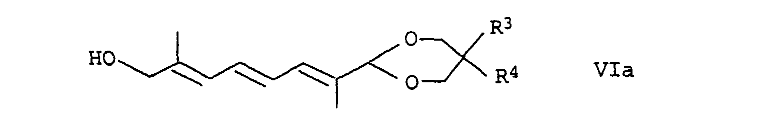

Der nächstliegende Stand der Technik ist die Oxidation eines C₁₀-Dimethyl-Acetal-alkohols VII zum korrespondierenden Aldehyd.

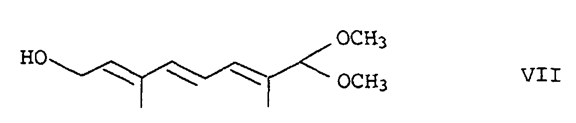

Wie in Helv. Chim. Acta 1966, 49, 369 beschrieben, wird diese Reaktion mit einem großen Überschuß Mangandioxid in 60-stündiger Reaktionszeit durchgeführt. Nachteilig an diesem Verfahren ist neben der extrem langen Reaktionszeit und der geringen Ausbeute die Verwendung eines mehrfach molaren Überschusses an Oxidationsmittel verbunden mit einem nicht unerheblichen Aufwand für die Feststoffabtrennung und die Entsorgung der Mangansalze.

Für die Oxidation von VI zu I im Schritt c) des erfindungsgemäßen Verfahrens kommen u.a. die in DE-A-3705785, DE-A-4440286, DE-A-4440287 sowie in EP-A-0 718 283 genannten Oxidationsverfahren zur Überführung von Polyenalkoholen in Polyenaldehyde in Frage. Unter den oben erwähnten ökonomischen, ökologischen und verfahrenstechnischen Gesichtspunkten werden jedoch katalytische Methoden bevorzugt. Als Katalysatoren hierfür sind u.a. Ruthenium-Verbindungen wie Tetrapropylammonium-perruthenat, Tris (triphenyl-phosphin)-Ruthenium (II) chlorid oder 1,5-Cyclooctadien-Ruthenium (II) chlorid in Mengen von 2 bis 4 mol-% in Gegenwart von mindestens stöchiometrischen Mengen an 4-Methylmorpholin-N-oxid als Co-Oxidans einsetzbar (siehe J. Chem. Soc. Chem. Commun. 1987, 1625).

Die katalytische Oxidation von VI zu I erfolgt bevorzugt mit einem Gemisch, enthaltend 2,2,6,6-Tetramethyl-piperidin-1-oxyl/ Kupfer(I)chlorid/Dimethylformamid/Sauerstoff oder mit einem Gemisch, enthaltend 4-Hydroxy-2,2,6,6-tetramethyl-piperidin-1-oxyl/Kupfer(I)chlorid/Dimethylformamid/Sauerstoff in Dimethylformamid als Solvens durchgeführt. Nähere Einzelheiten zur Oxidation finden sich u.a. in DE-A-3705785 und EP-A-0 718 283.

DE-A-3705785 und EP-A-0 718 283 beschreiben Oxidationen von Allylalkoholen, die ß-ständig zur Alkoholgruppe mit Methyl substituiert sind, also keine sterische Hinderung aufweisen. Es war daher unerwartet und überraschend, daß die Übertragung dieser Reaktion auf Allylalkohole der Formel VI mit α-ständigem Methyl-Substituenten in vollständigem und glattem Umsatz den korrespondierenden α,β-ungesättigten Aldehyd liefert.

Das erfindungsgemäße Verfahren löst die eingangs geschilderte Aufgabenstellung durch einen Prozeß, der
a) von technisch gut verfügbaren Edukten ausgeht,
b) in einer nur dreistufigen Synthese mit einfachen Verfahrensschritten in hoher Gesamtausbeute zum angestrebten Produkt führt, ohne daß
c) Zwischenstufen durch aufwendige Verfahrensschritte gereinigt werden müssen.

Gegenstand der Erfindung sind auch Acetal-ester der allgemeinen Formel Vb 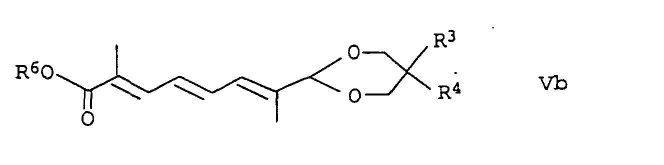 in der R⁶ C₁-C₄-Alkyl bedeutet und die Substituenten R³ und R⁴ gemeinsam entweder Wasserstoff oder Methyl bedeuten können.

Gegenstand der Erfindung sind außerdem Acetal-alkohole der allgemeinen Formel VI 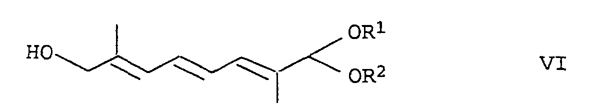 in der die Substituenten unabhängig voneinander folgende Bedeutung haben:
- R¹ und R²: C₁-C₈-Alkyl oder gemeinsam mit den Sauerstoffatomen und dem Kohlenstoffatom, an denen sie gebunden sind einen 1,3-Dioxolan- oder 1,3-Dioxan-Ring der folgenden Strukturen, 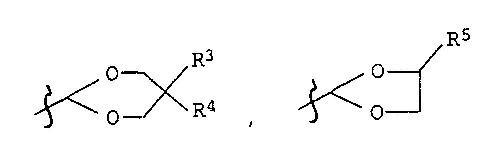 in denen R³ und R⁴ sowie R⁵ jeweils unabhängig voneinander Wasserstoff oder C₁-C₄-Alkyl bedeuten können.

Bevorzugt sind Acetal-alkohole der Formel VIa 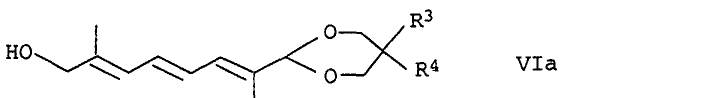 in der die Substituenten R³ und R⁴ gemeinsam entweder Wasserstoff oder Methyl bedeuten können.

Zur Definition der Substituenten R¹ bis R⁶ - in der allgemeinen und bevorzugten Ausführungsform - sei auf die eingangs genannten Erläuterungen hingewiesen.

Anhand des folgenden Beispiels soll das erfindungsgemäße Verfahren näher erläutert werden.

### Beispiel 1

### a) Herstellung des C₁₀-Acetal-esters (Vc)

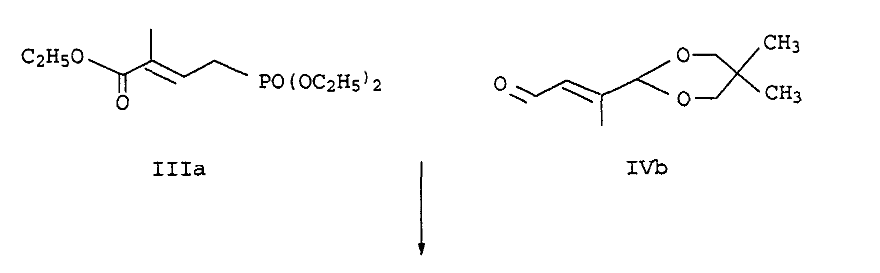

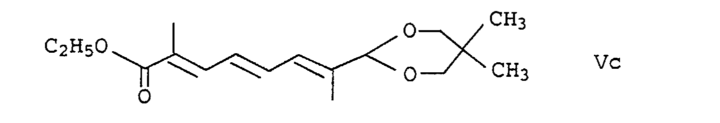

145 g (0,55 mol) C₅-Esterphosphonat IIIa und 103 g (0,55 mol) C₅-Acetal-aldehyd IVb wurden in 1250 ml Methylenchlorid vorgelegt. Bei 0°C wurde innerhalb von 1 h eine Lösung von 43 g (0,60 mol) Na-Ethylat in 163 g Ethanol zugegeben. Man rührte 1 h bei 0°C und anschließend 1 h bei Raumtemperatur nach. Anschließend wurde das Reaktionsgemisch zuerst mit 60 g 10%ige wäßrige Essigsäure und danach mit 500 ml halbkonzentrierter Kochsalzlösung versetzt. Die organische Phase wurde abgetrennt, zweimal mit einem Gemisch aus jeweils 250 ml halbkonzentrierter Kochsalzlösung und 50 ml 10%iger wäßriger Essigsäure und einmal mit 250 ml Wasser gewaschen, über Natriumsulfat getrocknet und am Rotationsverdampfer eingeengt. Der ölige Rückstand wurde im Vakuum bei 1 mbar und 50°C getrocknet. Man erhielt 163 g des rohen C₁₀-Acetal-esters Vc in quantitativer Ausbeute. Das Rohprodukt wurde direkt in die nächste Stufe (Reduktion zum Alkohol) eingesetzt.

### b) Herstellung des C₁₀-Acetal-alkohols VIc

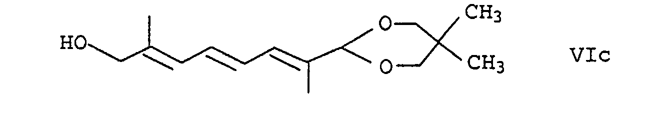

369 g roher C₁₀-Acetal-ester Vc (hergestellt gemäß Ausführungsbeispiel 1a) wurden in einem Gemisch aus 1000 ml Toluol und 1000 ml Tetrahydrofuran gelöst. Unter Kühlung auf 0°C bis -2°C wurden innerhalb von 1 h 400 ml einer 70%igen toluolischen Lösung von Natrium-dihydrido-bis(2-methoxyethoxy)-aluminat-Lösung zugetropft. Man rührte 2,5 h bei 0°C nach. Dann tropfte man bei 0°C zunächst 600 ml eines Ethanol/Hexan-Gemisches (V/V, 40:60) und anschließend 300 ml 15%ige wäßrige Natronlauge und 1200 ml n-Hexan zu. Man erhielt zwei klare Phasen. Die wäßrige Phase wurde abgetrennt und einmal mit 300 ml Toluol nachextrahiert. Der toluolische Extrakt wurde mit der organischen Phase vereinigt. Die vereinigten organischen Phasen wurden mehrmals mit je 400 ml gesättigter Kochsalzlösung gewaschen, bis die Wasserphase einen pH-Wert von 9 aufwies, über Natriumsulfat getrocknet und am Rotationsverdampfer eingeengt. Der ölige Rückstand wurde im Vakuum bei 1 mbar und 50°C getrocknet. Man erhielt 315 g des rohen C₁₀-Acetal-alkohols VIc in quantitativer Ausbeute. Das Rohprodukt wurde direkt in die nächste Stufe (Oxidation zum Aldehyd) eingesetzt.

### c) Herstellung des C₁₀-Monoacetal-aldehyds Ia

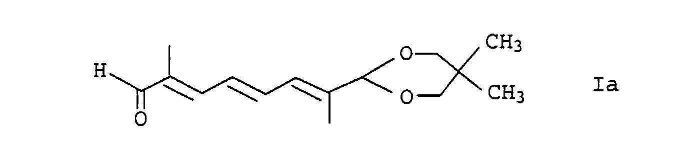

178 g roher C₁₀-Acetal-alkohol VIc (hergestellt gemäß Ausführungsbeispiel 1b) wurden in 750 ml Dimethylformamid vorgelegt. Nach Zugabe von 4,76 g 2,2,6,6-Tetramethyl-piperidin-N-Oxyl und 3,1 g Kupfer(I)chlorid wurde bei einer Temperatur von 25°C bis 30°C über einen Begasungsrührer Sauerstoffgas eingeleitet, bis die Umsetzung nach GC-Kontrolle vollständig war. In der Lösung vorhandener Sauerstoff wurde durch kurzes Begasen mit Stickstoff ausgetrieben.

Zur Aufarbeitung wurde das Gemisch mit 1000 ml Methyl-tert. Butylether und 1000 ml halbkonzentrierter wäßriger Kochsalzlösung versetzt. Die wäßrige Phase wurde abgetrennt und zweimal mit je 500 ml eines Gemisches aus Methyl-tert. Butylether und n-Hexan (v/v, 1 : 1) nachextrahiert. Die organischen Phasen wurden vereinigt, dreimal mit je 300 ml halbkonzentrierter wäßriger Kochsalzlösung gewaschen, über Natrium-sulfat getrocknet und am Rotationsverdampfer eingeengt. Der Rückstand wurde im Vakuum bei 1 mbar und 50°C getrocknet.

Aus zwei gleichartigen Ansätzen wurden zusammen 332 g roher C₁₀-Monoacetal-aldehyd Ia als Eindampfrückstand gewonnen.

Dieser rohe C₁₀-Monoacetal-aldehyd wurde bei 60°C in 2000 ml eines 1:1-Gemisches aus Diisopropylether/n-Hexan gelöst und heiß über Kieselgel als Filtriermittel filtriert. Das Filtrat wurde langsam abgekühlt und mehrere Stunden im Eiswasserbad ausgerührt. Das Kristallisat wurde über eine Glasfilternutsche abgesaugt, mit kaltem 1:1-Gemisch Dissopropylether/Hexan gewaschen und im N₂-Strom getrocknet. Man erhielt 145 g Erstkristallisat (53 % d. Th.) mit einem Schmelzpunkt von 84,5 bis 85°C und einer Reinheit nach GC von 98,2 %.

Mutterlauge und Waschlaugen wurden eingedampft. Der Rückstand wurde in der Hitze in einem Gemisch aus 300 ml Diisopropylether und 900 ml n-Hexan gelöst. Die Lösung wurde langsam abgekühlt und über Nacht bei 0°C ausgerührt. Das Kristallisat wurde wie oben beschrieben isoliert. Die Auswaage des Zweitkristallisats betrug 23 g (8,4 % d. Th.); Reinheit nach GC: 97,7 %.

Der Eindampfrückstand der Mutterlauge des Zweitkristallisats wurde mittels Flash-Chromatographie an Kieselgel (Eluat: Cyclohexan/Essigester 2:1) gereinigt. Der aufgereinigte C₁₀-Monoacetalaldehyd wurde wie oben beschrieben aus Diisopropylester/n-Hexan 1:3 kristallisiert. Auswaage Drittkristallisat: 46 g (16,7 % d. Th.).

Gesamtauswaage: 214 g kristallreiner C₁₀-Monoacetal-aldehyd Ia (77,8 % d. Th.)

## Patentansprüche

1. Verfahren zur Herstellung von 2,7-Dimethyl-2,4,6-octatrienalmonoacetalen der allgemeinen Formel I, 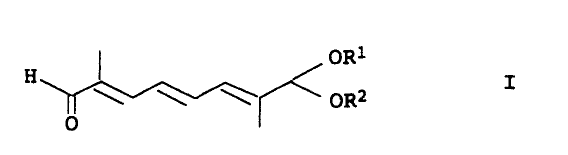 in der die Substituenten R¹ und R² unabhängig voneinander für C₁-C₈-Alkyl stehen oder gemeinsam mit den Sauerstoffatomen und dem Kohlenstoffatom, an denen sie gebunden sind einen 1,3-Dioxolan- oder 1,3-Dioxan-Ring der folgenden Strukturen bilden können, 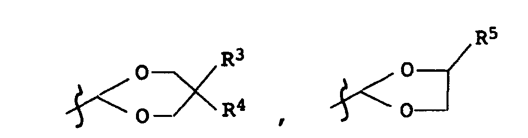 in denen R³ und R⁴ sowie R⁵ jeweils unabhängig voneinander Wasserstoff oder C₁-C₄-Alkyl bedeuten können,
**dadurch gekennzeichnet, daß** man
a) ein Esterphosphoniumsalz der allgemeinen Formel II oder ein Esterphosphonat der allgemeinen Formel III, 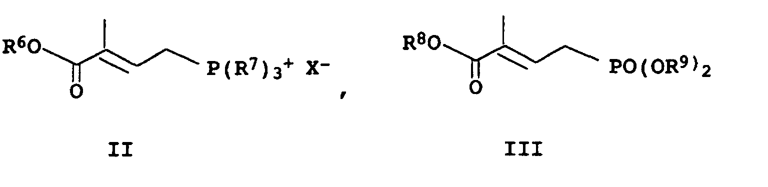 in denen die Substituenten unabhängig voneinander folgende Bedeutung haben:
R⁶ C₁-C₈-Alkyl;
R⁷ Aryl;
X⁻ ein Anionenäquivalent einer anorganischen oder organischen Säure;
R⁸ und R⁹ C₁-C₈-Alkyl;
mit einem Aldehyd der Formel IV 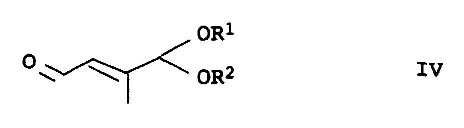 in einer Wittig- oder Wittig-Horner-Reaktion zu einem Acetal-ester der allgemeinen Formel V kondensiert, 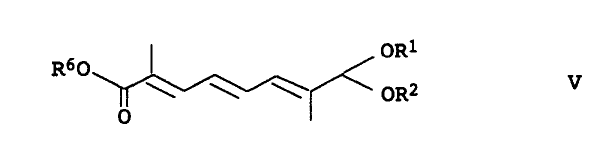 in denen die Substituenten R¹, R² und R⁶ der Verbindungen IV bzw. V die oben genannte Bedeutung haben,
b) den Ester der Formel V zu einem Acetal-alkohol der allgemeinen Formel VI reduziert, 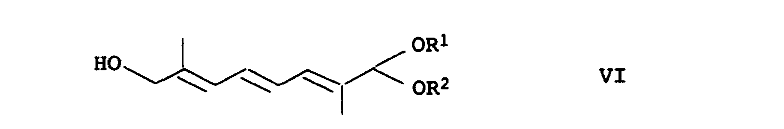 und
c) den Alkohol zu 2,7-Dimethyl-2,4,6-octatrienal-monoacetalen der allgemeinen Formel I oxidiert.

2. Verfahren nach Anspruch 1, **dadurch gekennzeichnet, daß** man im Verfahrensschritt a) ein Esterphosphonat der allgemeinen Formel III verwendet, in der die Substituenten R⁸ und R⁹ unabhängig voneinander C₁-C₃-Alkyl bedeuten.

3. Verfahren nach einem der Ansprüche 1 oder 2, **dadurch gekennzeichnet, daß** man im Verfahrensschritt a) einen Aldehyd der Formel IVa verwendet, 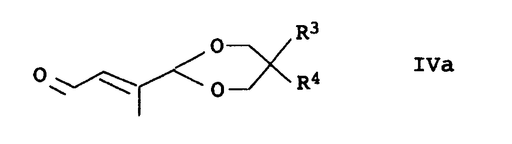 in der die Substituenten R³ und R⁴ gemeinsam entweder Wasserstoff oder Methyl bedeuten können.

4. Verfahren nach einem der Ansprüche 1 bis 3, **dadurch gekennzeichnet, daß** man im Verfahrensschritt b) die Reduktion der Esterfunktion mit einer Natrium-Aluminium-Hydridverbindung durchführt.

5. Verfahren nach Anspruch 4, **dadurch gekennzeichnet, daß** man im Verfahrensschritt b) die Reduktion der Esterfunktion mit Natrium-dihydro-bis-(2-methoxyethoxy)-aluminat durchführt.

6. Verfahren nach einem der Ansprüche 1 bis 5, **dadurch gekennzeichnet, daß** man im Verfahrensschritt c) die Oxidation des Alkohols VI zum entsprechenden Aldehyd I mit einem Gemisch, enthaltend 2,2,6,6-Tetramethyl-piperidin-1-oxyl/Kupfer(I)chlorid/Dimethylformamid/Sauerstoff oder mit einem Gemisch, enthaltend 4-Hydroxy-2,2,6,6-tetramethyl-piperidin-1-oxyl/Kupfer(I)chlorid/Dimethylformamid/Sauerstoff durchführt.

7. Acetal-ester der Formel Vb, 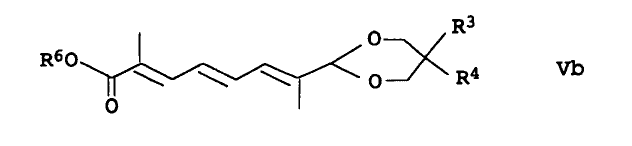 in der R⁶ C₁-C₄-Alkyl bedeutet und die Substituenten R³ und R⁴ gemeinsam entweder Wasserstoff oder Methyl bedeuten können.

8. Acetal-alkohole der allgemeinen Formel VI 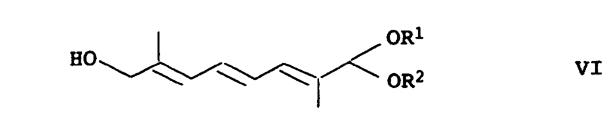 in der die Substituenten unabhängig voneinander folgende Bedeutung haben:
R¹ und R² C₁-C₈-Alkyl oder gemeinsam mit den Sauerstoffatomen und dem Kohlenstoffatom, an denen sie gebunden sind einen 1,3-Dioxolan- oder 1,3-Dioxan-Ring der folgenden Strukturen, 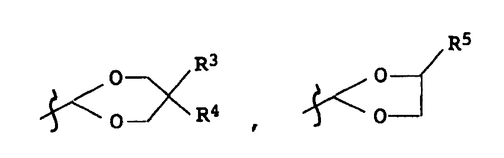 in denen R³ und R⁴ sowie R⁵ jeweils unabhängig voneinander Wasserstoff oder C₁-C₄-Alkyl bedeuten können.

9. Acetal-alkohole der Formel VIa nach Anspruch 8, 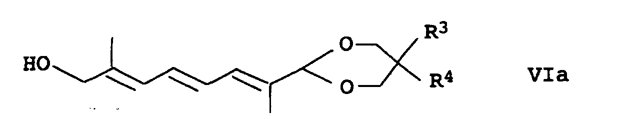 in der die Substituenten R³ und R⁴ gemeinsam entweder Wasserstoff oder Methyl bedeuten können.

## Claims

1. A process for preparing 2,7-dimethyl-2,4,6-octatrienal monoacetals of the general formula I, 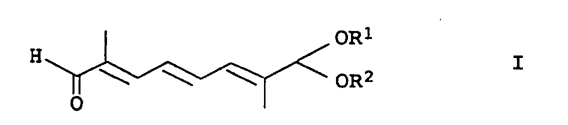 in which the substituents R¹ and R² may, independently of one another, be C₁-C₈-alkyl or, together with the oxygen atoms and the carbon atom to which they are bonded, form a 1,3-dioxolane or 1,3-dioxane ring of the following structures 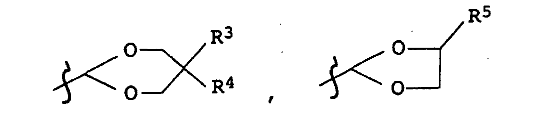 in which R³ and R⁴, and R⁵ may each, independently of one another, be hydrogen or C₁-C₄-alkyl,
which comprises
a) condensing an ester phosphonium salt of the general formula II or an ester phosphonate of the general formula III, 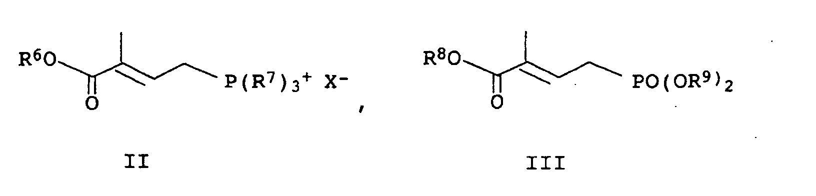 in which the substituents have, independently of one another, the following meaning:
R⁶ C₁-C₈-alkyl;
R⁷ aryl;
X⁻ an anion equivalent of an inorganic or organic acid;
R⁸ and R⁹ C₁-C₈-alkyl;
with an aldehyde of the formula IV 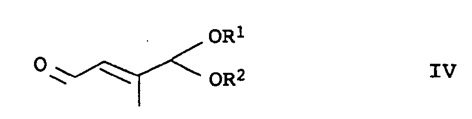 in a Wittig or Wittig-Horner reaction to give an acetal ester of the general formula V, 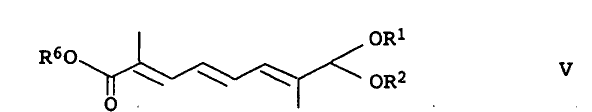 in which the substituents R¹, R² and R⁶ in the compounds IV and V have the abovementioned meaning,
b) reducing the ester of the formula V to an acetal alcohol of the general formula VI, 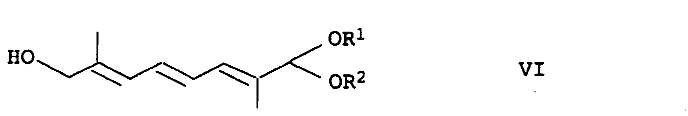 and
c) oxidizing the alcohol to 2,7-dimethyl-2,4,6-octatrienal monoacetals of the general formula I.

2. A process as claimed in claim 1, wherein an ester phosphonate of the general formula III in which the substituents R⁸ and R⁹ are, independently of one another, C₁-C₃-alkyl is used in process step a).

3. A process as claimed in either of claims 1 or 2, wherein an aldehyde of the formula IVa 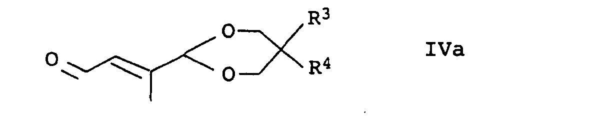 in which the substituents R³ and R⁴ may jointly be either hydrogen or methyl is used in process step a).

4. A process as claimed in any of claims 1 to 3, wherein the ester function is reduced in process step b) with a sodium aluminum hydride compound.

5. A process as claimed in claim 4, wherein the ester function is reduced in process step b) with sodium dihydrobis(2-methoxyethoxy)aluminate.

6. A process as claimed in any of claims 1 to 5, wherein the alcohol VI is oxidized to the corresponding aldehyde I in process step c) with a mixture comprising 2,2,6,6-tetramethylpiperidin-1-oxyl/copper (I) chloride/dimethylformamide/oxygen or with a mixture comprising 4-hydroxy-2,2,6,6-tetramethylpiperidin-1-oxyl/copper(I) chloride/dimethylformamide/oxygen.

7. An acetal ester of the formula Vb 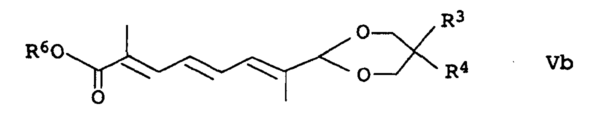 in which R⁶ is C₁-C₄-alkyl, and the substituents R³ and R⁴ may jointly be either hydrogen or methyl.

8. An acetal alcohol of the formula VI 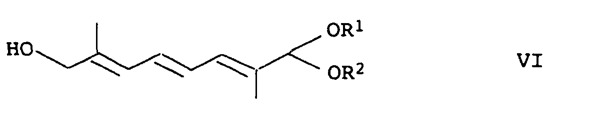 in which the substituents have, independently of one another, the following meaning:
R¹ and R² C₁-C₈-alkyl or together with the oxygen atoms and the carbon atoms to which they are bonded a 1,3-dioxolane or 1,3-dioxane ring of the following structures, 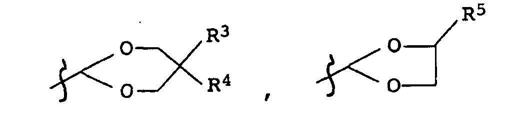 in which R³ and R⁴, and R⁵ may each, independently of one another, be hydrogen or C₁-C₄-alkyl.

9. An acetal alcohol as claimed in claim 8, of the formula VIa 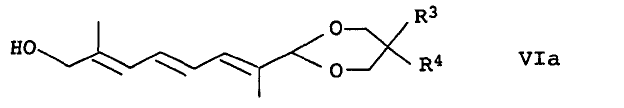 in which the substituents R³ and R⁴ may jointly be either hydrogen or methyl.

## Revendications

1. Procédé pour la préparation de monoacétals de 2,7-diméthyl-2,4,6-octatriénal de formule générale I, 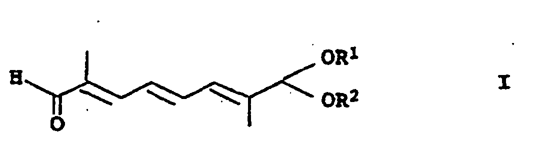 dans laquelle les substituants R¹ et R² désignent indépendamment l'un de l'autre un alkyle en C₁-C₈ ou peuvent former ensemble avec les atomes d'oxygène et l'atome de carbone auxquels ils sont liés un cycle 1,3-dioxolanne ou 1,3-dioxanne ayant les structures suivantes, 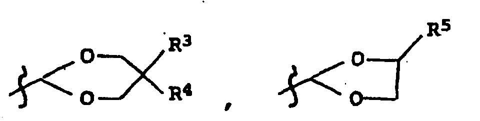 dans lesquelles R³ et R⁴ ainsi que R⁵ peuvent représenter à chaque fois indépendamment l'un de l'autre l'hydrogène ou un alkyle en C₁-C₄,
**caractérisé par le fait que**
a) on condense un sel d'esterphosphonium de formule générale II ou un esterphosphonate de formule générale III, 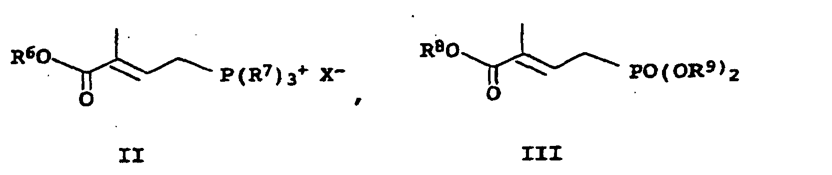 dans lesquelles les substituants ont indépendamment l'un de l'autre la signification suivante:
R⁶ alkyle en C₁-C₈;
R⁷ aryle;
X⁻ un équivalent d'anions d'un acide inorganique ou organique;
R⁸ et R⁹
alkyle en C₁-C₈;
avec un aldéhyde de formule IV 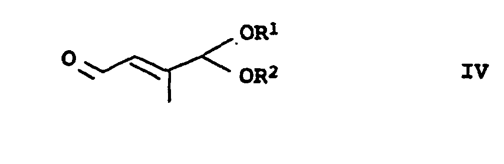 dans une réaction de Wittig ou de Wittig-Horner pour former un acétal-ester de formule générale V, 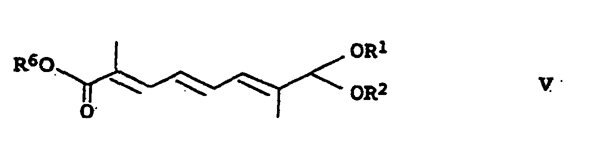 dans lesquelles les substituants R¹, R² et R⁶ des composés respectivement IV et V ont la signification indiquée plus haut,
b) on réduit l'ester de formule V pour former un acétal-alcool de formule générale VI, 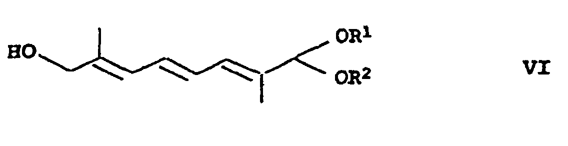 et
c) on oxyde l'alcool en monoacétals de 2,7-diméthyl-2,4,6-octatriénal de formule générale 1.

2. Procédé selon la revendication 1, **caractérisé par le fait qu'**on utilise dans l'étape opératoire a)un esterphosphonate de formule générale III, dans laquelle les substituants R⁸ et R⁹ désignent indépendamment l'un de l'autre un alkyle en C₁-C₃.

3. Procédé selon l'une des revendications 1 ou 2, **caractérisé par le fait qu'**on utilise dans l'étape opératoire a) un aldéhyde de formule générale IVa, 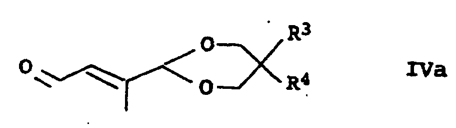 dans laquelle les substituants R³ et R⁴ peuvent représenter ensemble soit l'hydrogène soit le méthyle.

4. Procédé selon l'une des revendications 1 à 3, **caractérisé par le fait qu'**on effectue dans l'étape opératoire b) la réduction de la fonction ester avec un composé d'hydrure de sodium et d'aluminium.

5. Procédé selon la revendication 4, **caractérisé par le fait qu'**on effectue dans l'étape opératoire b) la réduction de la fonction ester avec du dihydro-bis-(2-méthoxyéthoxy)-aluminate de sodium.

6. Procédé selon l'une des revendications 1 à 5, **caractérisé par le fait qu'**on effectue dans l'étape opératoire c) l'oxydation de l'alcool VI en l'aldéhyde I correspondant avec un mélange contenant du 2,2,6,6-tétraméthylpipéridine-1-oxyle/chlorure de cuivre(I)/diméthylformamide/oxygène ou avec un mélange contenant du 4-hydroxy-2,2,6,6-tétraméthylpipéridine-1-oxyle/chlorure de cuivre(I)/diméthylformamide/oxygène.

7. Acétal-ester de formule Vb, 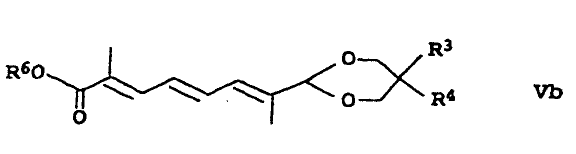 dans laquelle R⁶ désigne un alkyle en C₁-C₄ et les substituants R³ et R⁴ peuvent désigner ensemble soit l'hydrogène soit le méthyle.

8. Acétal-alcools de formule générale VI 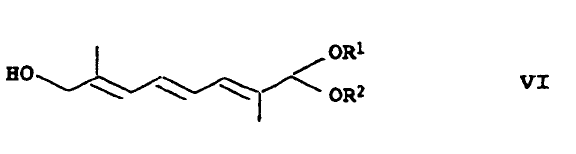 dans laquelle les substituants ont indépendamment l'un de l'autre la signification suivante:
R¹ et R² alkyle en C₁-C₈ ou ensemble avec les atomes d'oxygène et l'atome de carbone auxquels ils sont liés un cycle 1,3-dioxolanne ou 1,3-dioxanne ayant les structures suivantes, 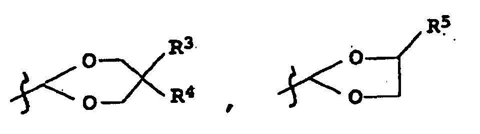 dans lesquelles R³ et R⁴ ainsi que R⁵ peuvent représenter à chaque fois indépendamment l'un de l'autre l'hydrogène ou un alkyle en C₁-C₄.

9. Acétal-alcools de formule VIa selon la revendication 8, 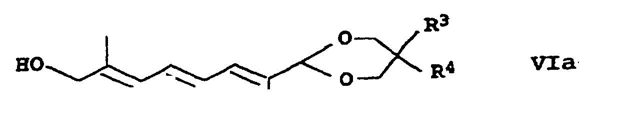 dans laquelle les substituants R³ et R⁴ peuvent représenter ensemble soit l'hydrogène soit le méthyle.
